(19) Europäisches Patentamt
European Patent Office
Office européen des brevets

(11) Veröffentlichungsnummer: **0 406 165 A2**

# (12) EUROPÄISCHE PATENTANMELDUNG

(21) Anmeldenummer: **90810449.0**

(51) Int. Cl.[5]: **C07C 315/04**

(22) Anmeldetag: **19.06.90**

(30) Priorität: **28.06.89 CH 2394/89**

(43) Veröffentlichungstag der Anmeldung: **02.01.91 Patentblatt 91/01**

(84) Benannte Vertragsstaaten: **BE CH DE ES FR GB IT LI**

(71) Anmelder: **CIBA-GEIGY AG**
**Klybeckstrasse 141**
**CH-4002 Basel(CH)**

(72) Erfinder: **Aeschlimann, Peter**
**Sandweg 16**
**CH-4123 Allschwil(CH)**

## (54) Verfahren zur Alkylierung aromatischer Amine.

(57) Die Erfindung betrifft ein Verfahren zur Herstellung von Verbindungen der Formel

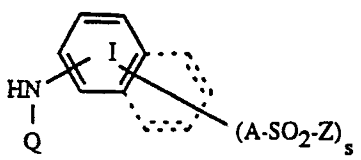
(1),

worin Q gegebenenfalls substituiertes $C_1$-$C_6$-Alkyl, A die direkte Bindung oder ein Brückenglied, s die Zahl 1 oder 2, Z ein Rest der Formel $-CH_2CH_2OH$, $-CH=CH_2$ oder $-CH_2CH_2-Y$, und Y eine Abgangsgruppe ist, und der Benzol- oder Naphthalinkern I weitersubstituiert sein kann, welches dadurch gekennzeichnet ist, dass man Verbindungen der Formel

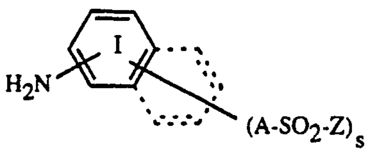
(2)

mit Verbindungen der Formel
Q-OH (3)
worin A, s, Z und Q die unter Formel (1) angegebenen Bedeutungen haben, in Gegenwart von Hydrierungskatalysatoren umsetzt, und gegebenenfalls weitere Umwandlungsreaktionen anschliesst.

Die nach dem erfindungsgemässen Verfahren erhaltenen Verbindungen eignen sich als Zwischenprodukte zur Herstellung von Reaktivfarbstoffen.

EP 0 406 165 A2

## VERFAHREN ZUR ALKYLIERUNG AROMATISCHER AMINE

Die Erfindung betrifft ein neues Verfahren zur Herstellung N-alkylierter aromatischer Amine, welche einen $\beta$-Oxäthylsulfonyl-Rest enthalten, aus den entsprechenden nichtalkylierten Aminen, indem man die Aminogruppe in Gegenwart von Hydrierungskatalysatoren mit einem Alkohol alkyliert.

Zur Herstellung monoalkylierter aromatischer Amine sind zahlreiche Verfahren in der Literatur bekannt. Nachteil der meisten Alkylierungsmethoden ist sowohl die unbefriedigende Ausbeute als auch die geringe Selektivität bei der Herstellung monoalkylierter Produkte.

Überraschenderweise erlaubt das erfindungsgemässe Verfahren die Herstellung N-monoalkylierter aromatischer Amine, welche einen $\beta$-Oxäthylsulfonyl-Rest enthalten, aus den entsprechenden nicht-alkylierten Aminen auf einfache Art und Weise, ohne die genannten Nachteile aufzuweisen.

Gegenstand der Erfindung ist somit ein Verfahren zur Herstellung von Verbindungen der Formel

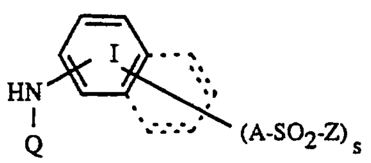

(1),

worin Q gegebenenfalls substituiertes $C_1$-$C_6$-Alkyl, A die direkte Bindung oder ein Brückenglied, s die Zahl 1 oder 2, Z ein Rest der Formel $-CH_2CH_2OH$, $-CH=CH_2$ oder $-CH_2CH_2-Y$, und Y eine Abgangsgruppe ist, und der Benzol- oder Naphthalinkern I weitersubstituiert sein kann, welches dadurch gekennzeichnet ist, dass man verbindungen der Formel

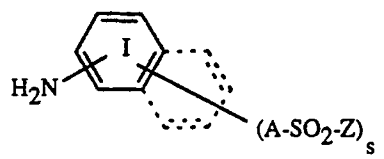

(2)

mit Verbindungen der Formel

Q-OH (3)

worin A, s, Z und Q die unter Formel (1) angegebenen Bedeutungen haben, in Gegenwart von Hydrierungskatalysatoren umsetzt, und gegebenenfalls weitere Umwandlungsreaktionen anschliesst.

Bei diesem Vorgehen erhält man überrachenderweise N-monoalkylierte aromatische Amine der Formel (1) in einer Reinheit von über 90 %. Ferner erhält man die Verbindungen der Formel (1) in höherer Ausbeute als nach den bisher üblichen Verfahren.

Die in dem erfindungsgemässen Verfahren verwendeten Amine der Formel (2) sind entweder Anilinderivate oder Naphthylaminderivate der Formeln

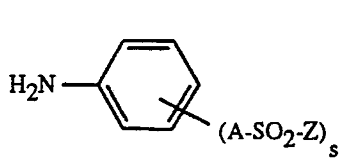

oder

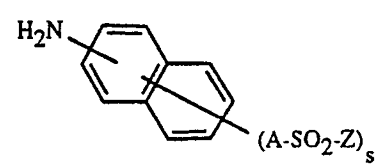

wobei der Benzolkern bzw. der Naphthalinkern weitersubstituiert sein kann und A, s und Z die unter Formel (1) angegebenen Bedeutungen haben. Als Substituenten des Benzolkern bzw. des Naphthalinkerns kommen z.B. die folgenden in Betracht: $C_1$-$C_4$-Alkyl, wie Methyl, Ethyl, Propyl, Isopropyl, Butyl, Isobutyl, sek.-Butyl und tert.-Butyl, $C_1$-$C_4$-Alkoxy, wie Methoxy, Ethoxy, Propoxy, Isopropoxy, sek.-Butoxy, tert.-Butoxy, Isobutoxy und n-Butoxy, Halogen, wie Fluor, Chlor und Brom und Sulfo. Bevorzugt sind die Substituenten Methyl, Ethyl, Methoxy, Ethoxy, Chlor, Brom und Sulfo; diese Substituenten sind insbesondere an den Benzolkern gebunden. Der Naphthalinkern ist vorzugsweise unsubstituiert oder durch eine Sulfogruppe substituiert.

Als $C_1$-$C_6$-Alkyl kommen für Q z.B. in Betracht: Methyl, Ethyl, n-Propyl, Isobutyl, sek.-Butyl, n-Butyl, n-Pentyl und n-Hexyl, sowie die entsprechenden Reste, die z.B. durch Hydroxy, Cyan, $C_1$-$C_4$-Alkoxy, wie z.B. Methoxy, Ethoxy, Isopropoxy, Propoxy und n-Butoxy, Halogen, wie Fluor, Chlor und Brom, Carboxy, Carbamoyl, $C_1$-$C_4$-Alkoxy-carbonyl, wie Methoxy-carbonyl und Ethoxycarbonyl, $C_1$-$C_4$-Alkylcarbonyloxy, wie Acetyloxy, Sulfo und Sulfamoyl substituiert sein können. Als Beispiele für derartige substituierte Reste seien genannt:

2-Hydroxyethyl, 2-Hydroxypropyl, 3-Hydroxypropyl, 2-Hydroxybutyl, 3-Hydroxybutyl, 4-Hydroxybutyl, 2,3-Dihydroxypropyl, 2,4-Dihydroxybutyl, Cyanmethyl, 2-Cyanethyl, 3-Cyanpropyl, Methoxymethyl, Ethoxymethyl, 2-Methoxyethyl, 2- Ethoxyethyl, 3-Methoxypropyl, 3-Ethoxypropyl, 2-Hydroxy-3-methoxypropyl, Chlormethyl, 2-Chlorethyl, 2-Bromethyl, Brommethyl, 3-Chlorpropyl, 4-Chlorbutyl, Carboxymethyl, 2-Carboxyethyl, 3-Carboxypropyl, 4-Carboxybutyl, 1 ,2-Dicarboxyethyl, Carbamoylmethyl, 2-Carbamoylethyl, 3-Carbamoylpropyl, Methoxycarbonylmethyl, 2-Methoxycarbonylethyl, 4-Methoxycarbonylbutyl, 4-Ethoxycarbonylbutyl, Methylcarbonyloxymethyl, 3-Methylcarbonyloxypropyl, 3-Ethylcarbonyloxypropyl, 4-Methylcarbonyloxybutyl, Sulfomethyl, 2-Sulfoethyl, 2-Sulfopropyl, Sulfamoylmethyl, 2-Sulfamoylethyl.

Als Brückenglied A kommen z.B. die folgenden Reste in Betracht: $-(CH_2)-_{1-2}$ und insbesondere $-CON(R_3)-(CH_2)_m$, $-(O)_0$ $-_1CH_2-CON(R_3)-(CH_2)_m$ oder $-N(R_3)-$ wie z.B. $-CONHCH_2CH_2-$, $-CH_2CONHCH_2CH_2-$, $-OCH_2CONHCH_2CH_2-$ und -N(H)-, wobei $R_3$ Wasserstoff, Methyl oder Ethyl bedeutet und m = 2,3,4,5 oder 6 ist. Falls A ein Brückenglied ist, ist der Rest $-SO_2Z$ an die $CH_2$-Gruppe der genannten Reste bzw. an das Stickstoffatom im Rest $-N(R_3)-$ gebunden.

Bei Y handelt es sich z.B. um einen unter alkalischen Bedingungen abspaltbaren anorganischen oder organischen Rest.

Beispiele für geeignete Reste Y sind:

$-OSO_3H$, $-SSO_3H$, $-OCOCH_3$, $-OCO-C_6H_5$, $OPO_3H_2$, -Cl, -Br, -F,

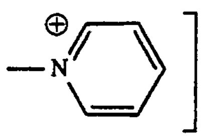
,
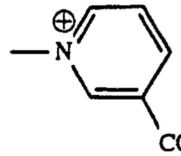
,
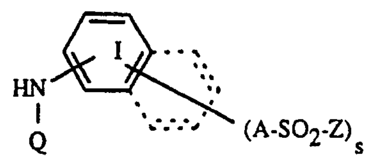
.

Vorzugsweise steht Y für die Gruppe $-OSO_3H$, $-OCOCH_3$, $-OPO_3H_2$ oder -Cl. Für s kommt die Zahl 1 oder 2 in Betracht. Vorzugsweise bedeutet s die Zahl 1.

Eine bevorzugte Ausführungsform des erfindungsgemässen Verfahrens ist dadurch gekennzeichnet, dass man als Verbindungen der Formel (2) Verbindungen der Formel (2a)

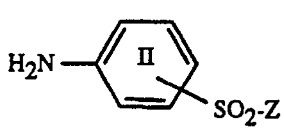
(2a)

verwendet, worin Z die unter Formel .(1) angegebene Bedeutung hat, und der Benzolkem II weitere Substituenten enthalten kann.

Eine weitere bevorzugte Ausführungsform des erfindungsgemässen Verfahrens ist dadurch gekennzeichnet, dass man Verbindungen der Formel

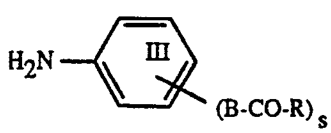
(2b)
.

verwendet, worin s die unter Formel (1) angegebene Bedeutung hat, B die direkte Bindung oder ein Rest der Formel $-(CH_2)_n$ oder $-O-(CH_2)_n$, n = 1 bis 6, R ein Rest der Formel

$- \underset{\underset{V}{|}}{N} -(alk)-CH_2-SO_2-Z$ (4a)

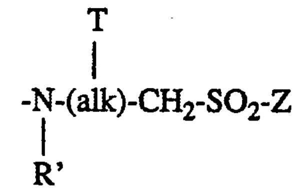

(4b),

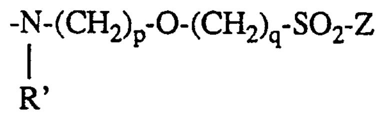

(4c),

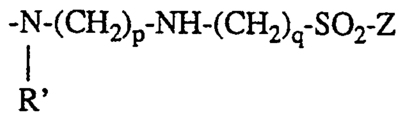

(4d),

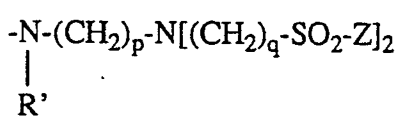

(4e) oder

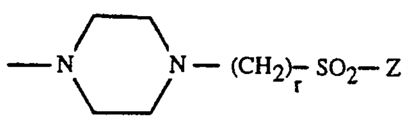

(4f),

worin R′ Wasserstoff oder $C_1$-$C_6$-Alkyl ist, (alk) einen $C_1$-$C_6$-Alkylenrest darstellt, T Wasserstoff, Halogen, Hydroxy, Sulfato, Carboxy, Cyano, $C_1$-$C_4$-Alkoxycarbonyl, Carbamoyl oder einen Rest -$SO_2$-Z bedeutet, V Wasserstoff, gegebenenfalls substituiertes $C_1$-$C_4$-Alkyl oder einen Rest der Formel -(alk)-$CH_2$-$SO_2$-Z, worin (alk) die zuvor angegebene Bedeutung hat, ist, Z die unter Formel (1) angegebene Bedeutung hat, und p, q und r unabhängig voneinander eine ganze Zahl von 1 bis 6 bedeuten, ist, und der Benzolkern III weitere Substituenten enthalten kann. Insbesondere bedeutet s in Formel (2b) die Zahl 1.

Bei R′ in den Formeln (4b) bis (4e) handelt es sich z.B. um Wasserstoff, Methyl, Ethyl, n-oder iso-Fropyl, n-, sec.- oder tert.-Butyl oder um einen geradkettigen oder verzweigten Pentyl- oder Hexylrest. Vorzugsweise steht R′ für Wasserstoff, Methyl oder Ethyl und besonders bevorzugt für Wasserstoff.

(alk) wie z.B. in den Formeln (4a) und (4b) bedeutet z.B. einen Methylen-, Ethylen, 1,3-Propylen-, 1,4-Butylen-, 1,5-Pentylen- oder 1,6-Hexylenrest oder dessen verzweigte Isomere. Bevorzugt ist für (alk) die Bedeutung eines $C_1$-$C_4$-Alkylenrestes und insbesondere die Bedeutung Methylen oder Ethylen.

p und q in den Formeln (4c), (4d) und (4e) stehen unabhängig voneinander bevorzugt für eine ganze Zahl von 1 bis 4; besonders bevorzugt bedeuten p und q jeweils die Zahl 2.

T steht vorzugsweise für Wasserstoff, Hydroxy, Sulfato, Acetyloxy, Carboxy, Methoxycarbonyl, Ethoxycarbonyl oder die Gruppe -$SO_2$-Z, wobei Z die zuvor angegebene Bedeutung hat; besonders bevorzugt ist T Wasserstoff oder ein Rest -$SO_2$-Z.

Ist V ein substituierter $C_1$-$C_4$-Alkylrest, so kann dieser z.B. durch Halogen, Hydroxy, Cyano, Carboxy, Sulfo, Sulfato, $C_1$-$C_4$-Alkoxy oder $C_1$-$C_4$-Alkoxycarbonyl substituiert sein.

Beispiele für substituierte $C_1$-$C_4$-Alkylreste sind: Carboxymethyl, β-Carboxyethyl, Methoxycarbonylmethyl, Ethoxycarbonylmethyl, β-Carboxyethyl, Methoxycarbonylmethyl, Ethoxycarbonylmethyl, β-Methoxyethyl, β-Ethoxyethyl, β-Chlorethyl, γ-Brompropyl, β-Hydroxyethyl, β-Hydroxybutyl, β-Cyanethyl, Sulfomethyl, β-Sulfoethyl, β-Sulfatoethyl.

Steht V für einen Rest der Formel Z-$O_2$S-$H_2$C-(alk)-, so kann sich dieser von dem in Formel (4a) vorhandenen zweiten Rest Z-$O_2$S-$H_2$C-(alk)- unterscheiden oder, vorzugsweise, mit letzterem übereinstimmen.

V bedeutet vorzugsweise Wasserstoff, Methyl, Ethyl oder die Gruppe $Z\text{-}O_2S\text{-}H_2C(alk)\text{-}$; insbesondere bevorzugt ist für V die Bedeutung Wasserstoff.

Beispiele für besonders bevorzugte Reste R sind:

$-NH-(CH)_2-SO_2-(CH_2)_2-OSO_3H$, $-NH-(CH_2)_2-SO_2-(CH_2)_2-OH$
$-NH-(CH_2)_3-SO_2-(CH_2)_2-OSO_3H$, $-NH-(CH_2)_3-SO_2-(CH_2)_2-OH$
$-NH-(CH_2)_2-O-(CH_2)_2-SO_2-(CH_2)_2-OSO_3H$, $-NH-(CH_2)_2-O-(CH_2)_2-SO_2-(CH_2)_2-OH$
$-N[-(CH_2)_2-SO_2-(CH_2)_2-OSO_3H]_2$, $-N[-(CH_2)_2-SO_2-(CH_2)_2-OH]_2$

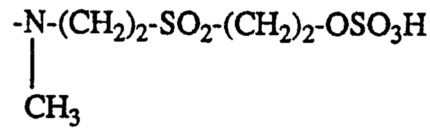 , 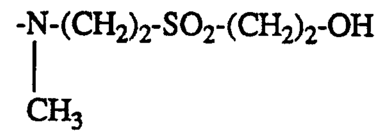

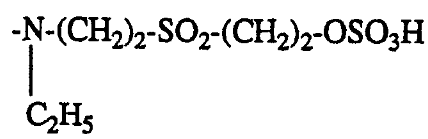 , 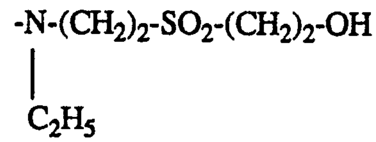

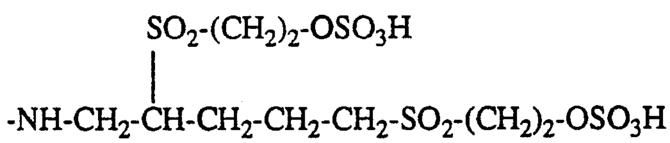 ,

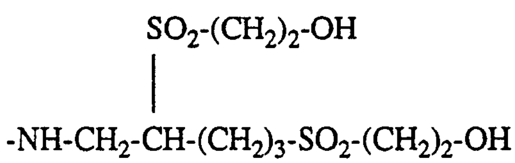

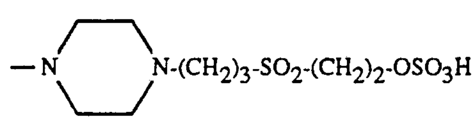 , 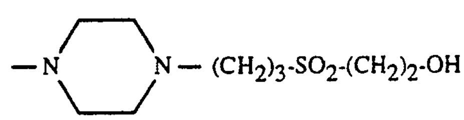 .

In dem erfindungsgemässen Verfahren geht man insbesondere von Verbindungen der Formel (2) bzw. (2a) oder (2b) aus, deren Benzolkern bzw. Naphthalinkern ausser der $H_2N$-Gruppe und dem Rest $-A-SO_2-Z$ keine weiteren Substituenten enthält.

Eine besonders bevorzugte Ausführungsform des erfindungsgemässen Verfahrens ist dadurch gekennzeichnet, dass man Verbindungen der Formel (3) verwendet, worin Q $C_1$-$C_4$-Alkyl, insbesondere Ethyl, ist.

Eine ganz besonders bevorzugte Ausführungsform des erfindungsgemässen Verfahrens ist dadurch gekennzeichnet, dass man Verbindungen der Formel (2), (2a) oder (2b) verwendet, worin Z $\beta$-Hydroxyethyl, $\beta$-Sulfatoethyl, $\beta$-Thiosulfatoethyl, $\beta$-Phosphatoethyl, $\beta$-Acetoxyethyl, $\beta$-Halogenethyl oder Vinyl ist. Insbesondere verwendet man Verbindungen der Formel (2), (2a) oder (2b), worin Z $\beta$-Hydroxyethyl ist. Die Ueberführung des Restes Z als $\beta$-Hydroxyethyl in einen anderen oben genannten Rest Z erfolgt insbesondere nach der Alkylierung.

Eine wichtige Ausführungsform des erfindungsgemässen Verfahrens zur Herstellung von Verbindungen der Formel

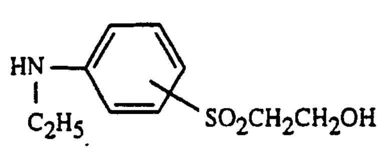 (5)

ist dadurch gekennzeichnet, dass man Verbindungen der Formel

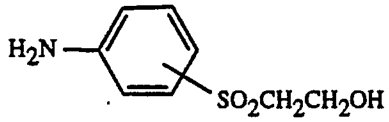

(6)

mit Ethanol in Gegenwart von Raney-Nickel umsetzt.

Eine weitere wichtige Ausführungsform des erfindungsgemässen Verfahrens zur Herstellung von Verbindungen der Formel

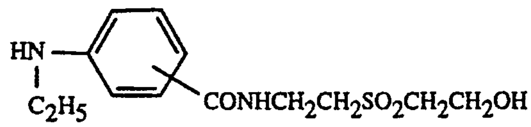

(7)

ist dadurch gekennzeichnet, dass man Verbindungen der Formel

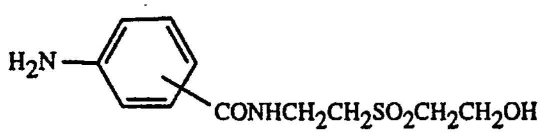

(8)

mit Ethanol in Gegenwart von Raney-Nickel umsetzt.

Das erfindungsgemässe Verfahren wird in Gegenwart von Hydrierungskatalysatoren ausgeführt. Als Hydrierungskatalysatoren kommen insbesondere Edelmetallkatalysatoren, wie z.B. Platin-, Palladium-, Nickel- und Kupferchromit-Katalysatoren in Betracht. Die erforderliche Reaktionstemperatur hängt im wesentlichen vom verwendeten Katalysator ab. Reaktionstemperaturen zwischen 50 und 250 °C haben sich als günstig erwiesen, vorzugsweise erfolgt die Umsetzung bei einer Temperatur zwischen 100 und 160 °C, insbesondere zwischen 120 und 140 °C, wobei sich als Hydrierungskatalysator ein Nickelkatalysator als besonders geeignet erwiesen hat. Ganz besonders bevorzugt wird in dem erfindungsgemässen Verfahren Raney-Nickel als Hydrierungskatalysator eingesetzt. Die Umsetzung kann unter Atmosphärendruck oder mit Überdruck erfolgen. Falls die Umsetzung mit Überdruck ausgeführt wird, liegt der Überdruck im allgemeinen zwischen 1 und 6 bar, insbesondere zwischen 2,5 und 4,5 bar. Vorzugsweise wird das erfindungsgemässe Verfahren wasserfrei ausgeführt. Die Reaktionszeit hängt im wesentlichen von der Aktivität des Hydrierungskatalysators, insbesondere Raney-Nickel, ab.

Eine bevorzugte Ausführungsform des erfindungsgemässen Verfahrens ist dadurch gekennzeichnet, dass man ohne Druck bei der Rückflusstemperatur der Verbindung der Formel (3) in Gegenwart von Raney-Nickel alkyliert.

Eine ebenfalls bevorzugte Ausführungsform des erfindungsgemässen Verfahrens ist dadurch gekennzeichnet, dass man Verbindungen der Formel (6) bzw. (8) mit Ethanol in Gegenwart von Raney-Nickel bei Temperaturen zwischen 120 und 140 °C und einem maximalen Überdruck von 4 bar umsetzt. In den Verbindungen der Formel (4) bzw. (8) ist die Position der $-NH_2$-Gruppe vorzugsweise in m- oder p-Stellung.

Die erfindungsgemäss erhaltenen Verbindungen der Formel (1), worin Z β-Hydroxyethyl ist, können in andere Verbindungen der Formel (1) umgewandelt werden, d.h. im Anschluss an die Alkylierung der Verbindung der Formel (2), worin Z β-Hydroxyethyl ist, erfolgt gegebenenfalls eine Umwandlungsreaktion, z.B. eine Veresterung oder Dehydratisierung.

So können Verbindungen der Formel (1), in denen die Gruppierung $-SO_2-Z$ eine β-Hydroxyethylsulfonylgruppe darstellt, durch Behandeln mit Sulfatierungsmitteln, Phosphorylierungsmitteln, Halogenierungsmitteln, Alkyl- oder Arylsulfonsäurehalogeniden, Alkyl- oder Arylcarbonsäurehalogeniden oder Alkyl- oder Arylcarbonsäureanhydriden in die entsprechenden Produkte übergeführt werden, in denen die Gruppierung $-SO_2-Z$ z.B. die Gruppierung $-SO_2-CH_2-CH_2-O-SO_3H$, $-SO_2-CH_2-CH_2-O-PO_3H_2$, $-SO_2-CH_2-CH_2$-Halogen, $-SO_2-CH_2-CH_2-O-CO-CH_3$ oder $-SO_2-CH_2-CH_2-O-CO-C_2H_5$ bedeutet. Die so erhaltenen Produkte können ihrerseits durch Behandeln mit alkalisch wirkenden Mitteln, wie beispielsweise Alkalihydroxid oder Alkalicarbonat, wie Natriumhydroxid oder Natriumcarbonat, in entsprechende Verbindungen übergeführt werden, in denen die Gruppierung $-SO_2-Z$ die Gruppierung $-SO_2-CH=CH_2$ bedeutet. Die so erhaltenen Produkte

können wiederum durch Umsetzung (Addition) mit Salzen der Thioschwefelsäure, wie Natriumthiosulfat, in Verbindungen übergeführt werden, in denen die Gruppierung $-SO_2-Z$ die Gruppierung $-SO_2-CH_2-CH_2-S-SO_3H$ bedeutet.

Geeignete Sulfatierungsmittel sind hierbei beispielsweise konzentrierte Schwefelsäure sowie Chlorsulfonsäure und Amidosulfonsäure oder andere Schwefeltrioxid abgebende Verbindungen. Geeignete Phosphorylierungsmittel sind hierbei beispielsweise konzentrierte Phosphorsäure, Pyro-, Meta- oder Polyphosphorsäurealkylester, Phosphoroxichlorid oder Gemische aus Phosphorsäure und Phosphor-(V)-oxid. Als Halogenierungsmittel können beispielsweise Thionylchlorid oder Thionylbromid verwendet werden.

Die Sulfatierung der Hydroxygruppe in einer Verbindung der Formel (1) erfolgt z.B. durch Umsetzung mit konzentrierter Schwefelsäure bei 0°C bis mässig erhöhter Temperatur. Die Sulfatierung kann auch durch Reaktion der Hydroxyverbindung mit zwei Äquivalenten Chlorsulfonsäure pro Hydroxygruppe in einem polaren organischen Lösungsmittel, wie beispielsweise N-Methylpyrrolidon, bei 10 bis 80°C erfolgen. Vorzugsweise erfolgt die Sulfatierung durch Eintragen der betreffenden Verbindung in Schwefelsäuremonohydrat bei Temperaturen zwischen 5 und 15°C. Die Einführung eines anderen Restes für Z in eine Verbindung der Formel (1) anstelle eines Halogenatoms oder der Sulfatogruppe beispielsweise einer Thiosulfato- oder Phosphatogruppe, erfolgt in an sich bekannter Weise.

Gegenstand der vorliegenden Erfindung ist somit auch ein Verfahren zur Herstellung von Verbindungen der Formel

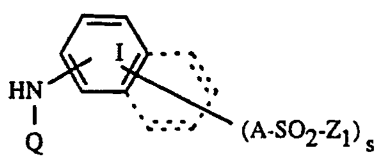

(9),

worin Q, s und A die unter Formel (1) angegebenen Bedeutungen haben, $Z_1$ ein Rest der Formel $-CH=CH_2$ oder $-CH_2CH_2-Y$, und Y eine Abgangsgruppe ist, und der Benzol- und Naphthalinkern I weitersubstituiert sein kann, welches dadurch gekennzeichnet ist, dass man Verbindungen der Formel

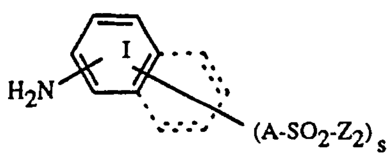

(10)

mit Verbindungen der Formel

Q-OH (3)

worin A, s und Q die unter Formel (1) angegebenen Bedeutungen haben, und $Z_2$ der Rest der Formel $-CH_2CH_2OH$ ist, in Gegenwart von Hydrierungskatalysatoren umsetzt, und anschliessend den Rest $Z_2$ in einen Rest $Z_1$ umwandelt, wobei die Umwandlung wie oben angegeben erfolgt. Für die Herstellung der Verbindung der Formel (9) aus der Verbindung der Formel (10) gelten die gleichen Bevorzugungen wie für die Herstellung der Verbindung der Formel (1) angegeben.

Eine bevorzugte Ausführungsform des erfindungsgemässen Verfahrens ist dadurch gekennzeichnet, dass man Verbindungen der Formel

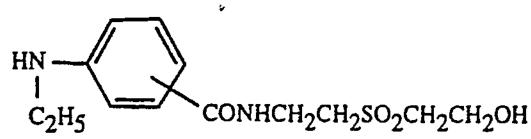

(7)

oder

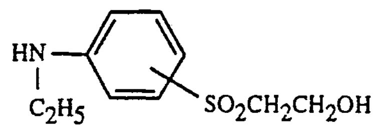

(5)

mit Sulfatierungsmitteln umsetzt.

Als Sulfatierungsmittel haben sich konzentrierte Schwefelsäure oder Chlorsulfonsäure in einem polaren organischen Lösungsmittel, oder Sulfaminsäure insbesondere in N-Methylpyrrolidon als besonders geeignet erwiesen.

Als Beispiele für Verbindungen der Formel (2) seien genannt:
3-(β-Hydroxyethylsulfonyl)-anilin, 4-(β-Hydroxyethylsulfonyl)-anilin, 2-(β-Hydroxyethylsulfonyl)-anilin, 3-Aminobenzoesäure-N-[β-(β′-hydroxyethylsulfonyl)]-ethylamid, 4-Aminobenzoesäure-N-[β-(β′-hydroxyethylsulfonyl)]-ethylamid.

Als Beispiele für Verbindungen der Formel (3) seien genannt:
Methanol, Ethanol, n-Propanol, n-Butanol, n-Pentanol, n-Hexanol.

Die Verbindungen der Formel (2), worin A die direkte Bindung ist, und die z.B. der Formel (2a) entsprechen, sind bekannt und können in an sich bekannter Weise erhalten werden.

Die Verbindungen der Formel (2), worin A ein Brückenglied ist, und die z.B. der Formel (2b) entsprechen, können in an sich bekannter Weise hergestellt werden, z.B. indem man eine Verbindung der Formel

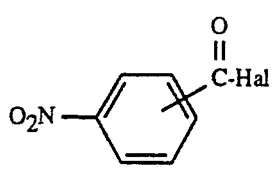

(11)

worin Hal Halogen, insbesondere Chlor, bedeutet, zunächst mit einem Amin der Formel

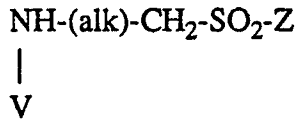

($4a_1$)

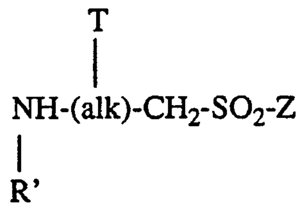

($4b_1$)

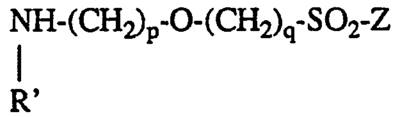

($4c_1$)

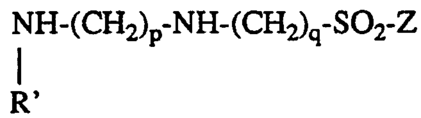

($4d_1$)

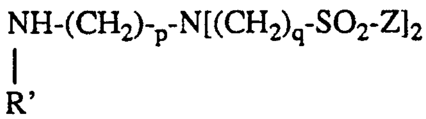

($4e_1$) oder

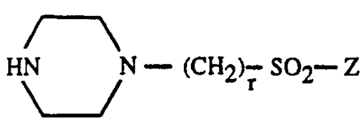

($4f_1$),

worin (alk), V, T, R′, p, q, r und Z jeweils die zuvor angegebene Bedeutung haben, insbesondere bedeutet Z β-Hydroxyethyl, umsetzt, die Nitrogruppe zur Aminogruppe reduziert und gegebenenfalls den Rest Z in einen anderen Rest Z verwandelt.

In Abänderung des oben beschriebenen Verfahrens kann anstelle der Nitroverbindung der Formel (11) auch die entsprechende Aminoverbindung eingesetzt werden, womit der Reduktionsschritt der Nitrogruppe entfällt. Die Nitroverbindungen der Formel (11) wie auch die entsprechenden Aminoverbindungen sind an sich bekannt oder können nach bekannten Methoden erhalten werden.

Eine weitere Variante des oben beschriebenen Verfahrens besteht darin, anstelle der Amine der Formeln ($4a_1$) bis ($4f_1$) geeignete Vorprodukte einzusetzen und diese nachträglich in die entsprechenden Amine umzuwandeln.

Geeignete Vorprodukte sind z.B. die den Formeln ($4a_1$) bis ($4f_1$) analogen Thioetheramine, die nach der Umsetzung mit einer Verbindung der Formel (11) in bekannter Weise zu den entsprechenden Sulfonen oxidiert werden können.

Es ist möglich, zunächst geeignete Halogenalkylamine mit dem Säurechlorid der Formel (11) reagieren zu lassen und die dabei erhaltenen Verbindungen mit 2-Mercaptoethanol und Natriumalkoholat in Alkohol zu den oben genannten Thioetheraminen umzusetzen, welche anschliessend wiederum zu den Sulfonen der Formeln ($4a_1$) bis ($4f_1$) oxidiert werden können.

Vorzugsweise werden solche Amine der Formeln ($4a_1$) bis ($4f_1$) bzw. deren Vorprodukte eingesetzt, welche eine Vorstufe des Reaktivrestes enthalten und dementsprechend Z z.B. einen Rest der Formel $HO-H_2C-H_2C-$ darstellt. Die Vorstufe des Reaktivrestes wird dann nachträglich wie weiter oben beschrieben in

die Endstufe überführt.

Die Amine der Formeln ($4a_1$) bis ($4f_1$) bzw. deren Vorprodukte sind z.B. aus der EP-A 210 951 bekannt oder lassen sich analog dazu herstellen.

Die Umsetzung (Kondensation) des Säurechlorids der Formel (11) mit den vorgenannten Aminen erfolgt zum Beispiel in wässrigem, wässrig-organischem oder organischem Medium bei einer Temperatur zwischen 0° und 120° C, vorzugsweise 10° bis 60° C, in Gegenwart von alkalischen säurebindenden Mitteln, z.B. Alkalimetallhydroxiden, -carbonaten oder -bicarbonaten.

Falls man von Vorprodukten der Amine der Formeln ($4a_1$) bis ($4f_1$) ausgeht, kann die Oxidation der Thioetherverbindungen zu den Sulfonen nach verschiedenen Methoden erfolgen, beispielsweise mit Wasserstoffperoxid mit oder ohne Zusatz von Wolfram-, oder Vanadinverbindungen als Katalysatoren, ferner mit Peressigsäure, Kaliumpermanganat oder Chromsäure, oder mit Chlor/Salzsäure je in wässrigem, wässrig-organischem oder organischem Medium.

Die Reduktion der Nitrogruppe zur Aminogruppe erfolgt in an sich bekannter Weise durch katalytische Hydrierung mit Pd/Kohlenstoff in Ethanol, Essigester oder Tetrahydrofuran bei Raumtemperatur bis etwa 40° C. Die Reduktion kann auch mit Fe/Essigsäure in wässriger Lösung durchgeführt werden.

Die so erhältlichen Carbonsäureamide, in denen die Gruppierung $-SO_2-Z$ eine $\beta$-Hydroxyethylsulfonylgruppe darstellt, können durch Behandeln mit Sulfatierungsmitteln, Phosphorylierungsmitteln, Halogenierungsmitteln, Alkyl- oder Arylsulfonsäurehalogeniden, Alkyl- oder Arylcarbonsäurehalogeniden oder Alkyl- oder Arylcarbonsäureanhydriden in die entsprechenden Produkte übergeführt werden, in denen die Gruppierung $-SO_2-Z$ z.B. die Gruppierung $-SO_2-CH_2-CH_2-O-SO_3H$, $-SO_2-CH_2-CH_2-O-PO_3H_2$, $-SO_2-CH_2-CH_2-$Halogen, $-SO_2-CH_2-CH_2-O-CO-CH_3$ oder $-SO_2-CH_2-CH_2-O-CO-C_2H_5$ bedeutet. Die so erhaltenen Produkte können ihrerseits durch Behandeln mit alkalisch wirkenden Mitteln, wie beispielsweise Alkalihydroxid oder Alkalicarbonat, wie Natriumhydroxid oder Natriumcarbonat, in entsprechende Verbindungen übergeführt werden, in denen die Gruppierung $-SO_2-Z$ die Gruppierung $-SO_2-CH=CH_2$ bedeutet. Die so erhaltenen Produkte können wiederum durch Umsetzung (Addition) mit Salzen der Thioschwefelsäure, wie Natriumthiosulfat, in Verbindungen übergeführt werden, in denen die Gruppierung $-SO_2-Z$ die Gruppierung $-SO_2-CH_2-CH_2-S-SO_3H$ bedeutet.

Die erfindungsgemäss erhaltenen Verbindungen der Formel (1) eignen sich als Zwischenprodukte bei der Herstellung von Reaktivfarbstoffen, indem z.B. zur Herstellung von bireaktiven Farbstoffen eine Verbindung der Formel (1), worin Z z.B. Vinyl bedeutet, mit einem Trihalogen-s-triazin kondensiert wird, und der erhaltene Dihalogen-s-triazinverbindung mit einem aminogruppenhaltigen Chromophor zu einem Reaktivfarbstoff mit einem Monohalogen-s-triazinrest umgesetzt wird.

Das erfindungsgemässe Verfahren weist gegenüber den bekannten Verfahren zur Herstellung der Verbindungen der Formel (1) folgende Vorteile auf:

Durch die selektive Monoalkylierung der aromatischen Amine der Formel (2) mit dem Alkylierungsmittel in Gegenwart von Hydrierungskatalysatoren kann die Umsetzung quantitativer erfolgen als es bisher möglich war.

Durch die höhere Reinheit der gemäss dem erfindungsgemässen Verfahren erhaltenen Verbindungen verglichen mit den auf übliche Weise hergestellt gleichen Verbindungen können Reaktivfarbstoffe mit reproduzierbar verbesserten Eigenschaften, insbesondere besserer Löslichkeit in Wasser erhalten werden.

Die nachfolgenden Beispiele dienen der Veranschaulichung der Erfindung. Darin sind die Teile Gewichtsteile und die Prozente Gewichtsprozente. Die Temperaturen sind in Celsiusgraden angegeben. Die Beziehung zwischen Gewichtsteilen und Volumenteilen ist dieselbe wie diejenige zwischen Gramm und Kubikzentimeter.

Beispiel 1:

136 Teile der Verbindung der Formel

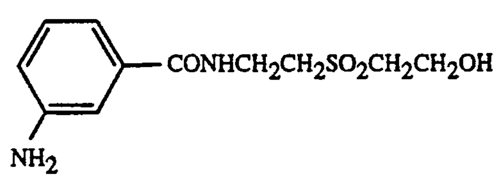

(100)

werden in 1000 Teilen Ethanol zusammen mit 50 Teilen Raney-Nickel in Ethanol suspendiert und während

24 Stunden bei 125° in einem Stahlautoklaven gerührt, wobei sich ein maximaler Druck von ca. 4 bar einstellt. Anschliessend wird das Reaktionsgemisch vom Raney-Nickel abfiltriert und in einem Eisbad gekühlt. Das Reaktionsprodukt der Formel

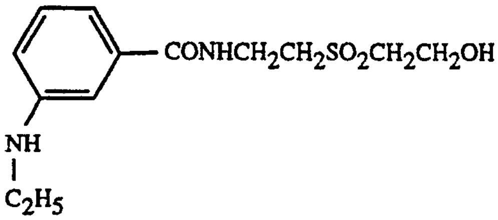

(101)

fällt als weisse Masse aus und wird kalt filtriert. Der Rückstand wird mit kaltem Ethanol gewaschen, abgepresst und dann getrocknet. Die Ausbeute beträgt 117 Teile (77 % der Theorie). Die Reinheit an monoethyliertem Produkt beträgt 92 %.

Beispiel 2:

100,5 Teile der Verbindung der Formel

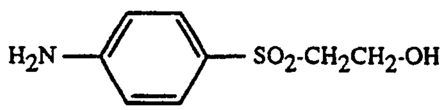

(102)

werden in 1500 Teilen Ethanol zusammen mit 50 Teilen Raney-Nickel in ethanolischer Suspension 36 Stunden bei 135° in einem Stahlautoklaven gerührt. Anschliessend wird vom Raney-Nickel abfiltriert und das Filtrat zur Trockne eingedampft. Man erhält 110 Teile der Verbindung der Formel

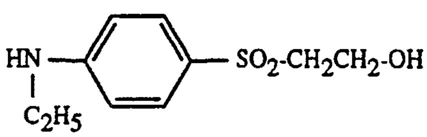

(103).

Die Reinheit an monethyliertem Produkt beträgt 94 %.

Wenn man wie oben angegeben verfährt und anstelle der Verbindung der Formel (102) eine äquimolare Menge 2-Amino-6-(β-hydroxyethylsulfonyl)-naphthalin verwendet, so erhält man als Reaktionsprodukt 2-(N-Ethylamino)-6-(β-hydroxyethylsulfonyl)-naphthalin.

Beispiel 3:

136 Teile der Verbindung der Formel

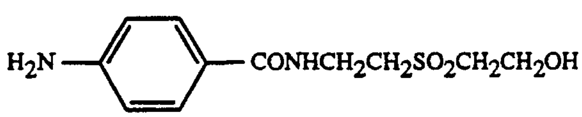

(104)

werden in 1000 Teilen Ethanol mit 50 Teilen Raney-Nickel in ethanolischer Suspension während 48 Stunden bei 135° gerührt. Anschliessend wird vom Raney-Nickel abfiltriert und das Reaktionsprodukt gekühlt. Das Reaktionsprodukt der Formel

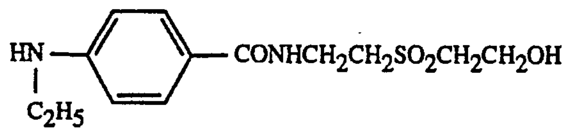

(105)

fällt als weisse Masse aus und wird kalt filtriert. Der Rückstand wird mit kaltem Ethanol gewaschen, abgepresst und dann getrocknet. Das erhaltene Produkt weist eine Reinheit an monoethyliertem Produkt von 90% auf.

Beispiel 4: Sulfatierung der Verbindung der Formel (101) aus Beispiel 1.

60 Teile der isolierten und getrockneten Verbindung der Formel (101) werden zusammen mit 29,1 Teilen Sulfaminsäure in 45 Teilen N-Methylpyrrolidon verrührt und drei Stunden bei einer Temperatur von 75-80° C gerührt. Die Sulfatierung erfolgt quantitativ. Das Reaktionsprodukt entspricht in Form der freien Säure der Formel

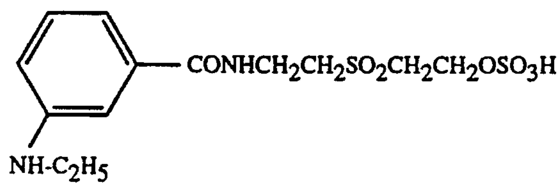

(106)

Das Reaktionsprodukt der Formel (106) kann ohne Isolation in Folgestufen eingesetzt werden.

Beispiel 5: Sulfatierung der Verbindung der Formel (103) aus Beispiel 2.

18,4 Teile der getrockneten Verbindung der Formel (103) werden bei 5-10° C in 90 Teilen Oleum (25%ig) eingetragen. Man erhöht die Temperatur auf 50° C und rührt eine Stunde.

Die klare Reaktionslösung ist einheitlich und frei von Ausgangsmaterial.

Nun wird bei 0° C auf Eis ausgetragen, mit 90 Teilen $CaCO_3$ ein pH-Wert von 1,5 und dann mit Sodalösung ein Wert von pH 5 eingestellt. Nach Filtration des Gipses wird die Mutterlauge zur Trockne eingeengt. Das Reaktionsprodukt entspricht in Form der freien Säure der Formel

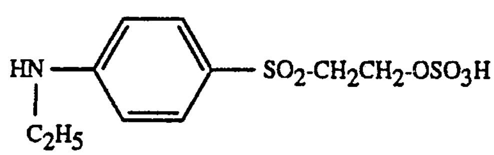

(107)

Wenn man wie in den Beispielen 1 bis 3 angegeben verfährt, und anstelle der Verbindungen der Formeln (100), (102) und (104) eine äquimolare Menge einer Verbindung der Formel

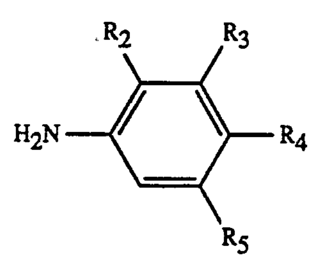

(108),

worin $R_2$, $R_3$, $R_4$ und $R_5$ die in der folgenden Tabelle angegebenen Bedeutungen haben, in einem Alkohol der Formel

Q-OH (109),

worin Q die in der folgenden Tabelle angegebene Bedeutung hat, in Gegenwart von Raney-Nickel bei 130-140 °C während 10 bis 20 Stunden und einem Druck zwischen 2 bis 6 bar umsetzt, so erhält man Verbindungen der Formel

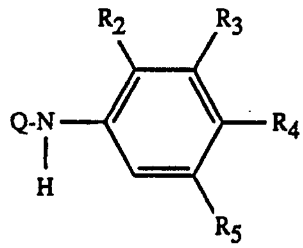

(110),

worin $R_2$, $R_3$, $R_4$, $R_5$ und Q die in der folgenden Tabelle angegebenen Bedeutungen haben.

Tabelle

| Beispiel | $R_2$ | $R_3$ | $R_4$ | $R_5$ | Q |
|---|---|---|---|---|---|
| 6 | -H | $-SO_2CH_2CH_2OH$ | -H | -H | $-C_2H_5$ |
| 7 | -H | $-SO_2CH_2CH_2OH$ | -H | -H | $n-C_3H_7$ |
| 8 | -H | $-SO_2CH_2CH_2OH$ | -H | -H | $n-C_4H_9$ |
| 9 | -H | -H | $-SO_2CH_2CH_2OH$ | -H | $n-C_3H_7$ |
| 10 | -H | -H | $-SO_2CH_2CH_2OH$ | -H | $n-C_4H_9$ |
| 11 | $-SO_2CH_2CH_2OH$ | -H | -H | -H | $-C_2H_5$ |
| 12 | $-CONH(CH_2)_2SO_2CH_2CH_2OH$ | -H | -H | -H | $-C_2H_5$ |
| 13 | $-CONH(CH_2)_2SO_2CH_2CH_2OH$ | -H | -H | -H | $n-C_3H_7$ |
| 14 | $-CONH(CH_2)_2SO_2CH_2CH_2OH$ | -H | -H | -H | $n-C_4H_9$ |
| 15 | -H | -H | $-CONH(CH_2)_2SO_2CH_2CH_2OH$ | -H | $n-C_3H_7$ |
| 16 | -H | -H | $-CONH(CH_2)_2SO_2CH_2CH_2OH$ | -H | $n-C_4H_9$ |
| 17 | -H | $-CONH(CH_2)_2SO_2CH_2CH_2OH$ | -H | $-CONH(CH_2)_2SO_2CH_2CH_2OH$ | $-C_2H_5$ |
| 18 | -H | $-CONH(CH_2)_2SO_2CH_2CH_2OH$ | -H | $-CONH(CH_2)_2SO_2CH_2CH_2OH$ | $n-C_3H_7$ |
| 19 | $-CH_3$ | -H | $-CONH(CH_2)_2SO_2CH_2CH_2OH$ | -H | $-C_2H_5$ |
| 20 | -Cl | -H | $-CONH(CH_2)_2SO_2CH_2CH_2OH$ | -H | $-C_2H_5$ |
| 21 | $-CH_3$ | -H | -H | $-CONH(CH_2)_2SO_2CH_2CH_2OH$ | $n-C_3H_7$ |
| 22 | -H | $-CON(CH_2CH_2SO_2CH_2CH_2OH)_2$ | -H | -H | $n-C_3H_7$ |
| 23 | -H | $-CON(CH_2CH_2SO_2CH_2CH_2OH)_2$ | -H | -H | $n-C_4H_9$ |
| 24 | -H | -H | $-CON(CH_2CH_2SO_2CH_2CH_2OH)_2$ | -H | $n-C_3H_7$ |
| 25 | -H | -H | $-CON(CH_2CH_2SO_2CH_2CH_2OH)_2$ | -H | $n-C_4H_9$ |
| 26 | -H | $-OCH_2CONHCH_2CH_2SO_2CH_2CH_2OH$ | -H | -H | $-C_2H_5$ |
| 27 | -H | $-CH_2SO_2CH_2CH_2OH$ | -H | -H | $-C_2H_5$ |
| 28 | -H | $-CH_2SO_2CH_2CH_2OH$ | -H | -H | $n-C_3H_7$ |
| 29 | -H | $-CH_2CH_2SO_2CH_2CH_2OH$ | -H | -H | $-C_2H_5$ |
| 30 | -H | -H | $-CH_2CH_2SO_2CH_2CH_2OH$ | -H | $-C_2H_5$ |

Die Sulfatierung der in der Tabelle angegebenen Hydroxyverbindungen erfolgt gemäss den in den Beispielen 4 und 5 angegebenen Bedingungen.

## Ansprüche

1. Verfahren zur Herstellung von Verbindungen der Formel

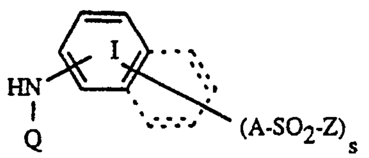

(1),

worin Q gegebenenfalls substituiertes $C_1$-$C_6$-Alkyl, A die direkte Bindung oder ein Brückenglied, s die Zahl 1 oder 2, Z ein Rest der Formel $-CH_2CH_2OH$, $-CH=CH_2$ oder $-CH_2CH_2$-Y, und Y eine Abgangsgruppe ist, und der Benzol- oder Naphthalinkern I weitersubstituiert sein kann, welches dadurch gekennzeichnet ist, dass man Verbindungen der Formel

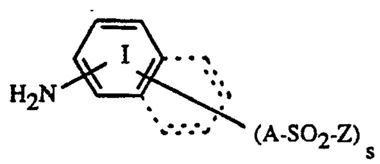

(2)

mit Verbindungen der Formel
Q-OH (3)
worin A, s, Z und Q die unter Formel (1) angegebenen Bedeutungen haben, in Gegenwart von Hydrierungskatalysatoren umsetzt, und gegebenenfalls weitere Umwandlungsreaktionen anschliesst.

2. Verfahren gemäss Anspruch 1, dadurch gekennzeichnet, dass man Verbindungen der Formel

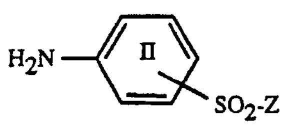

(2a)

verwendet, worin Z die in Anspruch 1 angegebene Bedeutung hat, und der Benzolkern II weitere Substituenten enthalten kann.

3. Verfahren gemäss Anspruch 1, dadurch gekennzeichnet, dass man Verbindungen der Formel

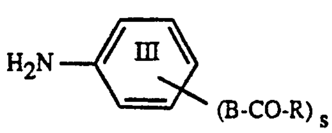

(2b)

verwendet, worin s die in Anspruch 1 angegebene Bedeutung hat, B die direkte Bindung oder ein Rest der Formel $-(CH_2)_n$ oder $-O(CH_2)_n$, n = 1 bis 6, R ein Rest der Formel

$$\begin{array}{l}-N\text{-}(alk)\text{-}CH_2\text{-}SO_2\text{-}Z\\ \;|\\ V\end{array}$$

(4a),

$$\begin{array}{l}\quad\; T\\ \quad\; |\\ -N\text{-}(alk)\text{-}CH_2\text{-}SO_2\text{-}Z\\ \;|\\ R'\end{array}$$

(4b),

$$\begin{array}{l}-N\text{-}(CH_2)_p\text{-}O\text{-}(CH_2)_q\text{-}SO_2\text{-}Z\\ \;|\\ R'\end{array}$$

(4c),

$$\begin{array}{l}-N\text{-}(CH_2)_p\text{-}NH\text{-}(CH_2)_q\text{-}SO_2\text{-}Z\\ \;|\\ R'\end{array}$$

(4d),

$$\begin{array}{l}-N\text{-}(CH_2)_p\text{-}N[(CH_2)_q\text{-}SO_2\text{-}Z]_2\\ \;|\\ R'\end{array}$$

(4e) oder

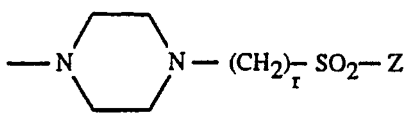

(4f),

worin R′ Wasserstoff oder $C_1$-$C_6$-Alkyl ist, (alk) einen $C_1$-$C_6$-Alkylenrest darstellt, T Wasserstoff, Halogen, Hydroxy, Sulfato, Carboxy, Cyano, $C_1$-$C_4$-Alkoxycarbonyl, Carbamoyl oder einen Rest $-SO_2$-Z bedeutet, V Wasserstoff, gegebenenfalls substituiertes $C_1$-$C_4$-Alkyl oder einen Rest der Formel -(alk)-$CH_2$-$SO_2$-Z, worin (alk) die zuvor angegebene Bedeutung hat, ist, Z die in Anspruch 1 angegebene Bedeutung hat, und p, q und r unabhängig voneinander eine ganze Zahl von 1 bis 6 bedeuten, ist, und der Benzolkern III weitere Substituenten enthalten kann.

4. Verfahren gemäss Anspruch 1, dadurch gekennzeichnet, dass man Verbindungen der Formel (3) verwendet, worin Q $C_1$-$C_4$-Alkyl ist.

5. Verfahren gemäss Anspruch 1, dadurch gekennzeichnet, dass man als Hydrierungskatalysator Raney-Nickel verwendet.

6. Verfahren gemäss einem der Ansprüche 1 und 4, dadurch gekennzeichnet, dass man als Verbindung der Formel (3) Ethanol verwendet.

7. Verfahren gemäss einem der Ansprüche 1, 2 und 3, dadurch gekennzeichnet, dass man Verbindungen der Formel (2), (2a) oder (2b) verwendet, worin Z $\beta$-Hydroxyethyl ist.

8. Verfahren gemäss einem der Ansprüche 1 bis 7 zur Herstellung von Verbindungen der Formel

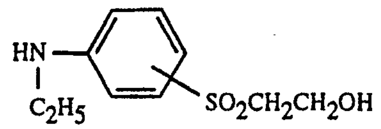

(5)

dadurch gekennzeichnet, dass man Verbindungen der Formel

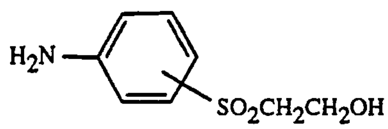

(6)

mit Ethanol in Gegenwart von Raney-Nickel umsetzt.

9. Verfahren gemäss einem der Ansprüche 1 bis 7 zur Herstellung von Verbindungen der Formel

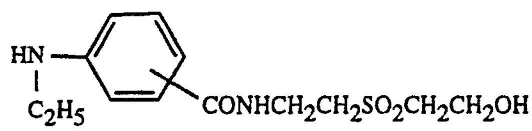

(7)

dadurch gekennzeichnet, dass man Verbindungen der Formel

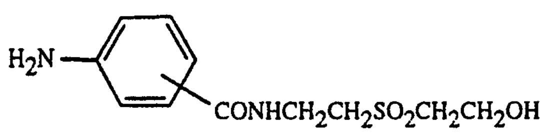

(8)

mit Ethanol in Gegenwart von Raney-Nickel umsetzt.

10. Verfahren gemäss einem der Ansprüche 2 und 3, dadurch gekennzeichnet, dass der Benzolkern II oder III unsubstituiert ist.

11. Verfahren gemäss Anspruch 1, dadurch gekennzeichnet, dass die Alkylierung der Verbindung der Formel (2) mit einer Verbindung der Formel (3) bei einer Temperatur zwischen 50 und 250 °C und gegebenenfalls unter Druck erfolgt.

12. Verfahren gemäss Anspruch 11, dadurch gekennzeichnet, dass die Alkylierung bei einer Temperatur zwischen 100 und 160 °C, insbesondere 120 und 140 °C, erfolgt.

13. Verfahren gemäss Anspruch 11, dadurch gekennzeichnet, dass man unter einem Druck von 1 bis 6 bar, insbesondere unter einem Druck von 2,5 bis 4,5 bar alkyliert.

14. Verfahren gemäss Anspruch 11, dadurch gekennzeichnet, dass man die Alkylierung unter Atmosphärendruck bei der Rückflusstemperatur der Verbindung der Formel (3) durchführt.

15. Verfahren gemäss Anspruch 1, dadurch gekennzeichnet, dass man die erhaltenen Verbindungen der Formel (1), worin Z β-Hydroxyethyl ist mit Veresterungsmitteln oder wasserabspaltenden Mitteln umsetzt.

16. Verfahren gemäss Anspruch 1 zur Herstellung von Verbindungen der Formel

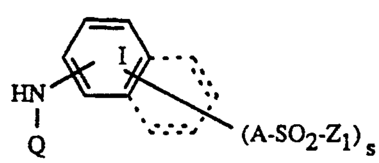

(9),

worin Q, s und A die in Anspruch 1 angegebenen Bedeutungen haben, $Z_1$ ein Rest der Formel $-CH=CH_2$ oder $-CH_2CH_2-Y$, und Y eine Abgangsgruppe ist, und der Benzol- und Naphthalinkern I weitersubstituiert sein kann, welches dadurch gekennzeichnet ist, dass man Verbindungen der Formel

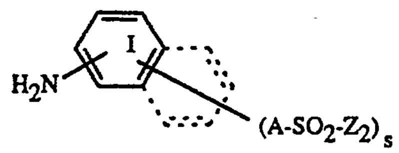

(10)

mit Verbindungen der Formel

Q-OH (3)

worin A, s und Q die in Anspruch 1 angegebenen Bedeutungen haben, und $Z_2$ der Rest der Formel $-CH_2CH_2OH$ ist, in Gegenwart von Hydrierungskatalysatoren umsetzt, und anschliessend den Rest $Z_2$ in einen Rest $Z_1$ umwandelt.

17. Verfahren gemäss Anspruch 16, dadurch gekennzeichnet, dass man Verbindungen der Formel

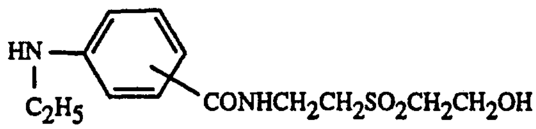

(7)

oder

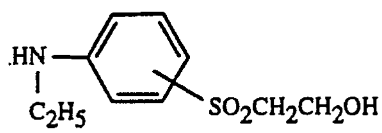

(5)

mit Sulfatierungsmitteln umsetzt.

18. Verfahren gemäss Anspruch 17, dadurch gekennzeichnet, dass man als Sulfatierungsmittel konzentrierte Schwefelsäure verwendet.

19. Verfahren gemäss Anspruch 17, dadurch gekennzeichnet, dass man als Sulfatierungsmittel Chlorsulfonsäure in einem polaren organischen Lösungsmittel verwendet.

20. Verfahren gemäss Anspruch 17, dadurch gekennzeichnet, dass man als Sulfatierungsmittel Sulfaminsäure in N-Methyl-pyrrolidon verwendet.

21. Verwendung der gemäss dem Verfahren nach Anspruch 16 erhaltenen Verbindungen zur Herstellung von Reaktivfarbstoffen.